(19)

Europäisches
Patentamt
European
Patent Office
Office européen
des brevets

(11) **EP 3 187 173 A1**

(12) **EUROPEAN PATENT APPLICATION**

(43) Date of publication:
05.07.2017 Bulletin 2017/27

(51) Int Cl.:
*A61K 9/14* (2006.01) *A61K 9/20* (2006.01)

(21) Application number: 17156237.4

(22) Date of filing: 18.04.2014

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 30.04.2013 JP 2013095725
30.09.2013 JP 2013204546

(62) Document number(s) of the earlier application(s) in
accordance with Art. 76 EPC:
14729061.3 / 2 838 513

(71) Applicant: Otsuka Pharmaceutical Co., Ltd.
Tokyo 101-8535 (JP)

(72) Inventors:
• **Yoshida, Haruka**
**Osaka, 540-0021 (JP)**
• **Taniguchi, Toshiaki**
**Osaka, 540-0021 (JP)**
• **Mukai, Tadashi**
**Osaka, 540-0021 (JP)**

(74) Representative: **Hoffmann Eitle**
**Patent- und Rechtsanwälte PartmbB**
**Arabellastraße 30**
**81925 München (DE)**

(54) **ORAL SOLID PREPARATION COMPRISING ARIPIPRAZOLE AND METHOD FOR PRODUCING ORAL SOLID PREPARATION COMPRISING ARIPIPRAZOLE**

(57)    An object of the present invention is to provide an oral solid preparation that can be produced in a simpler manner than conventional methods, that exhibits high bioavailability and high dissolubility even in persons having low stomach acid, and that can also ensure dissolubility after being allowed to stand for a certain period of time. Another object is to provide a simple method for producing the oral solid preparation.

The present invention relates to an oral solid preparation comprising, as an active ingredient, a finely milled powder obtained by milling a highly hygroscopic aripiprazole anhydrous crystal, which has a hygroscopicity such that the water content after being allowed to stand in a desiccator at a temperature of 60°C and a humidity of 100% for 24 hours is more than 0.40%, and a pharmaceutically acceptable carrier, the finely milled crystal having a mean particle size of 1 to 10 $\mu$m, wherein the mean particle size is the volume mean diameter as calculated from the particle size distribution measured by a laser scattering particle size distribution analyzer.

EP 3 187 173 A1

Printed by Jouve, 75001 PARIS (FR)

## Description

Technical Field

The present invention relates to an oral solid preparation comprising an aripiprazole hydrate or highly hygroscopic aripiprazole anhydrous crystal as an active ingredient, and a method for producing an oral solid preparation comprising an aripiprazole hydrate or highly hygroscopic aripiprazole anhydrous crystal as an active ingredient.

Background Art

Aripiprazole, i.e., 7-{4-[4-(2,3-dichlorophenyl)-1-piperazinyl]butoxy}-3,4-dihydrocarbostyril or 7-{4-[4-(2,3-dichlorophenyl)-1-piperazinyl]butoxy}-3,4-dihydro-2(1H)-quinolinone, is known as an atypical antipsychotic that is useful for the treatment of schizophrenia, and aripiprazole anhydrous crystals are also known (Patent Document 1: JP1990-191256A and Non-patent Document 1: Proceedings of the Fourth Japan-Korea Joint Symposium on Separation Technology).

The aripiprazole anhydrous crystals have high hygroscopicity, and are therefore problematic in various ways (paragraph [0006] of Patent Document 2). One such problem is that, due to their high hygroscopicity, aripiprazole anhydrous crystals are prone to hydration. Furthermore, compared to anhydrous crystals, hydrated crystals are low in bioavailability and dissolubility. In particular, hydrated crystals exhibit low bioavailability and dissolubility in people with low stomach acid. A high proportion of Asian persons have low stomach acid. In Japanese, who are of Asian ethnicity, 25% of people 60 years of age or older are said to have low stomach acid.

There are also other problems caused by high hygroscopicity. For example, Patent Document 2 describes, as problems, the variation in the amount of aripiprazole hydrate versus anhydrous aripiprazole from batch to batch; increased packaging cost due to concerns regarding moisture absorption during storage and handling; poor storage stability resulting from reduction of packaging cost; etc. (paragraph [0006] of Patent Document 2).

The aripiprazole anhydrous crystals disclosed in Patent Document 1 and Non-patent Document (NPL) 1 have problematically high hygroscopicity; however, such aripiprazole anhydrous crystals exhibit high bioavailability in persons having low stomach acid, and also have high dissolubility. Therefore, various research and analysis has been conducted on low hygroscopic aripiprazole anhydrous crystals.

As a result of such research and analysis, a novel low hygroscopic aripiprazole anhydrous crystal B that is resistant to hydration was successfully obtained from a novel aripiprazole hydrate A (Patent Document 2).

The low hygroscopic aripiprazole anhydrous crystal B can solve the above-mentioned various problems.

Patent Document 2 discloses that the novel aripiprazole anhydrous crystal B is produced by a method comprising milling a known aripiprazole hydrate (preferably monohydrate) to form a novel aripiprazole hydrate A, and heating the hydrate A to form a novel aripiprazole anhydrous crystal B.

Citation List

Patent Literature

PTL 1 JP1990-191256A (Japanese Patent No. 2608788)
PTL 2 Japanese Patent No. 3760264

Non Patent Literature

NPL 1 Satoshi Aoki et al., "Study on Crystal Transformation of Aripiprazole," Proceedings of the Fourth Japan-Korea Joint Symposium on Separation Technique (held on October 6 to 8, 1996), pages 937 to 940 (issued in Japan)

Summary of Invention

Technical Problem

However, the production process disclosed in the above Patent Document 2 comprises many production steps. If an oral solid preparation that exhibits high bioavailability and dissolubility in persons having low stomach acid can be produced in a simple manner, time and cost can be reduced.

Accordingly, an object of the present invention is to provide an oral solid preparation that can be produced in a manner simpler than conventional methods, that exhibits high bioavailability and high dissolubility even in persons having low stomach acid, and that can also ensure dissolubility after being allowed to stand for a certain period of time.

Another object of the present invention is to provide a simple method for producing an oral solid preparation that has high bioavailability and high dissolubility, and that can also ensure dissolubility after being allowed to stand for a certain period of time.

Solution to Problem

As a result of extensive research, the present inventors achieved the above objects by attempting the use of an aripiprazole hydrate crystal, which has low bioavailability and low dissolubility, and which had been considered inapplicable to medicine by persons skilled in the art.

Further, as a result of extensive research, the present inventors achieved the above objects by attempting the use of a highly hygroscopic aripiprazole anhydrous crystal which had been considered inapplicable to medicine by persons skilled in the art, i.e., an aripiprazole anhydrous crystal that is highly hygroscopic and therefore exhibits reduced dissolubility after storage for a certain period of time.

More specifically, a first embodiment of the present invention uses a finely milled crystal obtained by simply milling an aripiprazole hydrate crystal to a mean particle size in a specific range, and can thereby provide an oral solid preparation that has high bioavailability and high dissolubility equivalent to those of an oral solid preparation comprising aripiprazole anhydrous crystal B, and that can also ensure dissolubility after being allowed to stand for a certain period of time.

Further, the first embodiment of the present invention can provide a simple production method in which a finely milled crystal obtained by simply milling an aripiprazole hydrate crystal is directly used for the production of an oral solid preparation. For example, unlike the production process disclosed in Patent Document 2, the step of heating aripiprazole hydrate A for drying can be omitted.

The first embodiment of the present invention provides oral solid preparations according to items 1 to 7, and methods for producing the oral solid preparations.

Item 1. An oral solid preparation comprising, as an active ingredient, a finely milled crystal obtained by milling an aripiprazole hydrate crystal, and a pharmaceutically acceptable carrier, the finely milled crystal having a mean particle size of 15 $\mu$m or less.

Item 2. The oral solid preparation according to item 1, wherein the finely milled crystal has a mean particle size of 14 $\mu$m or less.

Item 3. The oral solid preparation according to item 1, wherein the finely milled crystal has a mean particle size of 10 $\mu$m or less.

Item 4. The oral solid preparation according to item 1, wherein the finely milled crystal has a mean particle size of 5 $\mu$m or less.

Item 5. The oral solid preparation according to any one of items 1 to 4, wherein the finely milled crystal has a mean particle size of 1 $\mu$m or more.

Item 6. A method for producing an oral solid preparation comprising the steps of:

(1) milling an aripiprazole hydrate crystal into a finely milled crystal having a mean particle size of 15 $\mu$m or less; and
(2) mixing the obtained finely milled crystal with a pharmaceutically acceptable carrier.

Item 7. The method for producing an oral solid preparation according to item 6, wherein the oral solid preparation is a tablet, and the method further comprises the step of (3) compressing the mixture obtained in step (2) into tablets.

A second embodiment of the present invention uses a finely milled aripiprazole crystal obtained by simply milling a highly hygroscopic aripiprazole anhydrous crystal to a mean particle size in a specific range, and can thereby provide an oral solid preparation that can ensure dissolubility after being allowed to stand for a certain period of time, and that has high bioavailability and dissolubility equivalent to those of an oral solid preparation comprising aripiprazole anhydrous crystal B.

Further, the second embodiment of the present invention can provide a simple production method in which a finely milled crystal obtained by simply milling a highly hygroscopic aripiprazole anhydrous crystal is directly used for the production of an oral solid preparation. For example, unlike the production process disclosed in Patent Document 2, the step of heating aripiprazole hydrate A for drying can be omitted.

The present invention provides oral solid preparations according to items 8 to 13, and methods for producing the oral solid preparations.

Item 8. An oral solid preparation comprising, as an active ingredient, a finely milled crystal obtained by milling a highly hygroscopic aripiprazole anhydrous crystal, and a pharmaceutically acceptable carrier, the finely milled crystal having a mean particle size of 10 $\mu$m or less. Item 9. The oral solid preparation according to item 8, wherein the finely milled crystal has a mean particle size of 8 $\mu$m or less.

Item 10. The oral solid preparation according to item 8, wherein the finely milled crystal has a mean particle size of 6 $\mu$m or less.

Item 11. The oral solid preparation according to any one of items 8 to 10, wherein the finely milled crystal has a mean particle size of 1 $\mu$m or more.

Item 12. A method for producing an oral solid preparation comprising the steps of:

(1') milling a highly hygroscopic aripiprazole anhydrous crystal into a finely milled crystal having a mean particle size of 10 $\mu$m or less; and

(2') mixing the obtained finely milled crystal with a pharmaceutically acceptable carrier.

Item 13. The method for producing an oral solid preparation according to item 12, wherein the oral solid preparation is a tablet, and the method further comprises the step of (3') compressing the mixture obtained in step (2') into tablets.

Advantageous Effects of Invention

The effect of the first embodiment of the present invention is as follows.

The first embodiment of the present invention can provide an oral solid preparation that can be produced in a simpler manner than conventional methods, that exhibits high bioavailability and high dissolubility even in people having low stomach acid, and that can also ensure dissolubility after being allowed to stand for a certain period of time.

The first embodiment of the present invention can further provide a simple method for producing an oral solid preparation that has high bioavailability and high dissolubility, and that can also ensure dissolubility after being allowed to stand for a certain period of time.

According to the first embodiment of the present invention, the mean particle size of the particles of the aripiprazole hydrate crystal as an active ingredient is set to a specific low range. This enables the production of an oral solid preparation that has high dissolubility without being affected by the external environment (e.g., external humidity), and whose dissolubility after being allowed to stand for a certain period of time is also not impaired.

Further, the oral solid preparation according to the first embodiment of the present invention does not require the use of an aluminum pouch when packing the pharmaceutical in a blister pack (a PTP film), or a desiccant when sealing the pharmaceutical in a bottle.

Additionally, when using the method for producing the oral solid preparation according to the first embodiment of the present invention, the oral solid preparation obtained by the method has high dissolubility without being affected by hygroscopicity of aripiprazole as an active ingredient, and dissolubility of the preparation in the form of a tablet is also not impaired even after being allowed to stand for a certain period of time. Therefore, for example, when the preparation is formed into tablets, usable methods are not only a wet granule compression method or like production methods that comprise the steps of first preparing granules having a controlled water content, and then drying to reduce the water content of the granules; tablets can also be easily produced by other compression methods, such as a direct compression method or a dry granule compression method.

The effect of the second embodiment of the present invention is as follows.

The second embodiment of the present invention can provide an oral solid preparation that can be produced in a simpler manner than conventional methods, that exhibits high bioavailability and high dissolubility even in people having low stomach acid, and that can also ensure dissolubility after being allowed to stand for a certain period of time.

The second embodiment of the present invention can further provide a simple method for producing an oral solid preparation that can ensure dissolubility after being allowed to stand for a certain period of time and that therefore has high bioavailability and high dissolubility.

According to the second embodiment of the present invention, the mean particle size of the particles of the highly hygroscopic aripiprazole anhydrous crystal as an active ingredient is set to a specific low range. This enables the production of an oral solid preparation which is not affected by the external environment (e.g., external humidity) and whose dissolubility after being allowed to stand for a certain period of time is not impaired.

Further, the oral solid preparation of the second embodiment of the present invention does not require the use of an aluminum pouch when packing the pharmaceutical in a blister pack (a PTP film), or a desiccant when sealing the pharmaceutical in a bottle.

Additionally, when using the method for producing the oral solid preparation according to the second embodiment of the present invention, the oral solid preparation obtained by the method is not affected by hygroscopicity of highly hygroscopic aripiprazole anhydrous crystal as an active ingredient; the oral solid preparation exhibits high dissolubility immediately after production, and dissolubility of the preparation after being allowed to stand for a certain period of time is also not impaired. Therefore, for example, when the preparation is formed into tablets, usable methods are not only a wet granule compression method or like production methods that comprise the steps of first preparing granules having a controlled water content, and then drying to reduce the water content of the granules; tablets can also be easily produced by other compression methods, such as a direct compression method or a dry granule compression method.

Brief Description of Drawings

Fig. 1 shows an X-ray diffraction pattern of aripiprazole anhydrous crystal of Lot No. 1.
Fig. 2 shows an X-ray diffraction pattern of aripiprazole anhydrous crystal of Lot No. 2.

Description of Embodiments

The expression "comprising" used herein includes the concepts of "comprising," "essentially consisting of," and "consisting of."

1. Oral solid preparation according to the first embodiment of the present invention

The oral solid preparation according to the first embodiment of the present invention comprises, as an active ingredient, an aripiprazole hydrate crystal having a mean particle size of 15 $\mu$m or less, and a pharmaceutically acceptable carrier.

The aripiprazole hydrate crystals that can be used in the present invention are not particularly limited, and any crystals may be used.

Examples of aripiprazole hydrate crystals include the hydrate crystals disclosed in Reference Example 3 and Example 1 of Japanese Patent No. 3760264.

Compared to aripiprazole anhydrous crystals, aripiprazole hydrate crystals generally exhibit lower dissolubility in a dissolution test for oral solid preparations, and their dissolubility tends to decrease with the passage of time. However, the present invention suppresses the decrease in dissolubility by setting the particle diameter of aripiprazole hydrate crystals to a specific mean particle size.

Another excellent effect achieved by the present invention is that dissolubility is ensured, even when the preparation formed into tablets is allowed to stand for a certain period of time.

The aripiprazole hydrate crystals that can be used in the present invention are not particularly limited. Specific examples thereof include crystalline forms disclosed in Patent Document 2, specifically, aripiprazole hydrate A, and aripiprazole Type III disclosed in Non-patent Document 1. These crystalline forms can be prepared by the methods disclosed in Patent Document 2 and Non-patent Document 1.

The mean particle size of aripiprazole hydrate crystals is about 15 $\mu$m or less, preferably about 14 $\mu$m or less, more preferably about 10 $\mu$m or less, and even more preferably about 5 $\mu$m or less. Setting the mean particle size of aripiprazole hydrate crystals to about 15 $\mu$m or less can impart high dissolubility to the obtained oral solid preparation, and ensure dissolubility after being allowed to stand for a certain period of time.

Setting the mean particle size to the above-mentioned specific range can achieve a dissolution rate of about 60% or more at pH 5 after 60 minutes (the dissolution rate that can maintain the maximum blood concentration ($C_{max}$)) in dissolution tests performed immediately after the production of the oral solid preparation, and after lapse of time.

The mean particle size of aripiprazole hydrate crystals is not particularly limited. However, the mean particle size is preferably about 1 $\mu$m or more, more preferably about 2 $\mu$m or more, and most preferably about 2.5 $\mu$m or more from the viewpoint of preventing an increase of the mean particle size due to reaggregation, reducing costs while not requiring excessive time to mill aripiprazole hydrate crystals, and facilitating handling. In particular, setting the mean particle size of aripiprazole hydrate crystals to 2 $\mu$m or more, more preferably about 2.5 $\mu$m or more, is preferable because it prevents dissolubility variation of aripiprazole hydrous crystals as an active ingredient, and can stably provide an oral solid preparation with excellent dissolubility.

More specifically, the aripiprazole hydrate crystals preferably have a mean particle size of about 1 to 15 $\mu$m (particularly about 2 to 15 $\mu$m, and more particularly about 2.5 to 15 $\mu$m), more preferably about 1 to 14 $\mu$m (particularly about 2 to 14 $\mu$m, and more particularly about 2.5 to 14 $\mu$m), even more preferably about 1 to 10 $\mu$m (particularly about 2 to 10 $\mu$m, and more particularly about 2.5 to 10 $\mu$m), and still even more preferably about 1 to 5 $\mu$m (particularly about 2 to 5 $\mu$m, and more particularly about 2.5 to 5 $\mu$m).

The term "mean particle size" refers to volume mean diameter as calculated from the particle size distribution measured by a laser scattering particle size distribution analyzer.

The particle size ratio of the specific particle size of particles can be calculated from particle size distribution measured by a laser scattering particle size distribution analyzer.

The mean particle size measurement by the above method can also be performed using a light microscope. The measurement can be performed in accordance with the method described in the Japanese Pharmacopoeia (for example, B. General Tests, 3.04 Particle Size Determination, 1. First Optical Microscopy, described in the Handbook of the Japanese Pharmacopoeia, 16th Edition, Health, Labor and Welfare Ministry, Notification 65 (March 24, 2011)). The mean particle size measured according to the method described in the Japanese Pharmacopoeia may be, for example, in the range of 3 to 20 $\mu$m.

The oral solid preparation preferably comprises an aripiprazole hydrate crystal in an amount of about 0.5 to 80 wt.%, and more preferably about 1 to 70 wt.%.

Examples of pharmaceutically acceptable carriers in the oral solid preparation of the present invention include diluents and excipients generally used in pharmaceuticals, such as fillers, extenders, binders, humectants, disintegrators, surfactants, and lubricants.

The pharmaceutical composition of the present invention may be in the form of a general pharmaceutical preparation. Examples of such forms include tablets, flash-melt tablets, pills, powders, granules, capsules, and the like.

When the preparation is formed into tablets, a wide variety of carriers conventionally known in this field can be used. Examples of such carriers include excipients such as lactose, sucrose, sodium chloride, glucose, urea, starch, xylitol, mannitol, erythritol, sorbitol, calcium carbonate, kaolin, crystalline cellulose, and silicic acid; binders such as water, ethanol, propanol, simple syrup, glucose solution, starch solution, gelatin solution, carboxymethyl cellulose, shellac, methylcellulose, potassium phosphate, and polyvinylpyrrolidones; disintegrators such as dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, and lactose; disintegration inhibitors such as sucrose, stearin, cacao butter, and hydrogenated oils; absorption enhancers such as quaternary ammonium base and sodium lauryl sulfate; humectants such as glycerol and starch; adsorbents such as starch, lactose, kaolin, bentonite, and colloidal silica; and lubricants such as purified talc, stearate, boric acid powder, and polyethylene glycol. Tablets can also be formed as tablets with ordinary coatings, if necessary. Examples of such tables include sugar-coated tablets, gelatin-coated tablets, enteric-coated tablets, film-coated tablets, double-layer tablets, and multilayer tablets.

When the preparation is formed into pills, a wide variety of carriers conventionally known in this field can be used. Examples of usable carriers include excipients such as glucose, lactose, starch, cacao butter, hydrogenated vegetable oil, kaolin, and talc; binders such as powdered gum arabic, powdered tragacanth, gelatin, and ethanol; disintegrators such as laminaran and agar; and the like.

Capsules are prepared according to ordinary methods by mixing an aripiprazole hydrate crystal with one or more of various carriers as exemplified above, and packing the mixture in hard gelatin capsules, soft capsules, hydroxypropylmethyl cellulose capsules (HPMC capsules), or the like.

If necessary, colorants, preservatives, perfumes, flavorings, sweeteners, and the like, as well as other drugs may be incorporated into the pharmaceutical composition.

When the solid oral preparation is formed into granules, a liquid (typically, water or an aqueous solution containing a binder) is added to a mixed powder of granulating ingredients comprising an aripiprazole hydrate crystal and a carrier. As the carrier, various carriers known in this field can be used. Examples of such carriers include excipients, disintegrators, disintegration inhibitors, humectants, absorption enhancers, adsorbents, lubricants, colorants, and the like. Specific examples thereof include those mentioned above.

The oral solid preparation of the present invention may have any dissolution rate that is about 60% or more at pH 5 after 60 minutes as measured by a dissolution test method. When the dissolution rate at pH 5 after 60 minutes is about 60% or more, the active ingredient in the oral solid preparation administered can be sufficiently absorbed into the body, and the maximum blood concentration ($C_{max}$) can be maintained. Therefore, a dissolution rate of about 60% or more is preferable.

The dissolution test method is in accordance with the dissolution test method (paddle method) in the Japanese Pharmacopoeia, Sixteenth Edition, and is a method as described below.

One tablet of an oral solid preparation is added to 500 ml of acetate buffer (pH 5.0), which is used as a test liquid, thereby obtaining a test solution. Using the test solution, a dissolution test is performed at a paddle speed of 75 rpm in accordance with the method 2 (paddle method) for dissolution test. As the acetate buffer, a solution prepared by adding water to 1.97 g of acetic acid and 9.15 g of sodium acetate trihydrate to make 1000 ml of solution is used.

A standard solution is prepared in the following manner. 0.05 g of a standard sample of aripiprazole is weighed accurately, and ethanol (95%) is added to make exactly 50 ml of solution. Then, 6 ml of this solution is weighed accurately, and the test liquid is added to make exactly 1000 ml of solution.

The test solutions and the standard solution are subjected to filtration using a filter having a pore size of 10 or 20 $\mu$m. Each of the filtrates is introduced to a spectrophotometer equipped with a flow cell (cell length: 10 mm), and tested by UV visible absorbance spectroscopy. The differences between the absorbance of each solution at a wavelength of 249 nm and the absorbance thereof at a wavelength of 325 nm, which are At10, At20, At30, At45, At60, and As, are determined. At10, At20, At30, At45, and At60 indicate differences between the absorbances of each test solution at 10, 20, 30, 45, and 60 minutes after the beginning of the dissolution test, respectively. As indicates a difference between the absorbances of the standard solution.

The dissolution rate (%) relative to the indicated amount of aripiprazole is determined by the following formula:

```
Dissolution Rate (%) relative to the indicated amount of
aripiprazole = Amount of the aripiprazole standard product
(mg) x At x As x 2
```

In the Formula, As indicates the difference between the absorbances of the standard solution, and At indicates At10, At20, At30, At45, or At60.

More specifically, the measurement of the dissolution rate by the above dissolution test method can be performed by the method shown in Test Example 1 of the Examples below.

The method for administering the oral solid preparation of the present invention is not particularly limited. The preparation can be orally administered by a method suitable for the patient's age, sex, and other conditions (e.g., severity of disease).

The dosage of the oral solid preparation of the present invention can be suitably selected according to the dose regimen, the patient's age, sex, and other conditions (e.g., severity of disease), etc. Generally, the preparation is administered in an amount such that the amount of aripiprazole, which is an active ingredient, is about 0.1 to 10 mg per kg of body weight per day. The amount of aripiprazole per tablet of the oral solid preparation is in the range of about 1 to 100 mg, and preferably about 1 to 30 mg.

The oral solid preparation of the present invention is effective in the prevention and treatment of schizophrenia, including intractable (drug-resistant, chronic) schizophrenia with cognitive impairment and intractable (drug-resistant, chronic) schizophrenia without cognitive impairment; anxiety, including mild anxiety; mania, including acute mania such as bipolar disorder acute mania; bipolar disorder; depression, including bipolar disorder depression; autism, Down's syndrome, attention deficit hyperactivity disorder (ADHD), Alzheimer's disease, Parkinson's disease, and like neurodegenerative diseases; panic, obsessive compulsive disorder (OCD), sleep disorder, sexual dysfunction, alcohol and drug dependency, vomiting, motion sickness, obesity, migraine, cognitive impairment, and like central nervous system diseases; and major depression, behavioral and psychological symptoms of dementia (BPSD), Tourette's syndrome, bulimia nervosa, and chronic pain/fibromyalgia/chronic fatigue syndrome.

2. Method for producing the oral solid preparation according to the first embodiment of the present invention

The method for producing an oral solid preparation according to the first embodiment of the present invention comprises the steps of (1) milling an aripiprazole hydrate crystal to obtain a finely milled crystal having a mean particle size of 15 $\mu$m or less, and (2) mixing the obtained finely milled crystal with a pharmaceutically acceptable carrier.

The finely milled crystal obtained by milling in step (1) can be directly subjected to the subsequent step (2). For example, without drying after milling, the finely milled crystal can be used to produce the oral solid preparation.

Examples of the aripiprazole hydrate crystal used in step (1) may be the same as the hydrate crystals mentioned above in "1. Oral solid preparation according to the first embodiment of the present invention."

The method for milling the aripiprazole hydrate crystal in step (1) is not particularly limited, and may be wet milling or dry milling. Various milling methods and mills can be used. The milling step can be performed by suitably selecting or suitably combining the conditions, devices, and methods that can produce an aripiprazole hydrate crystal having a mean particle size within the specific range of the present invention. From the viewpoint of ease of operation, dry milling is preferable.

When the milling in step (1) is performed by a dry milling method, examples of usable mills include jet mills, ball mills (e.g., Dyno mills), other low-energy mills (e.g., roller mills), and high-energy mills. Examples of high-energy mills include Netzsch mills, DC mills, planetary mills, and the like.

In particular, when a jet mill is used as a mill, the mean particle size of the milled aripiprazole hydrate crystal can be set by two types of air pressure ("forcing pressure" for feeding the powder to the milling chamber, and "milling pressure" of air directly sent to the milling chamber) and the "feed rate of the powder." Generally, as the air pressure is set high and the feed rate of the powder is set low, milling strength increases and the particle size tends to be small.

When the milling in step (1) is performed by wet milling, usable mills include, for example, high-pressure homogenizers and bead mills.

Step (2) is a step of mixing the aripiprazole hydrate crystal milled in step (1), and a pharmaceutically acceptable carrier.

Examples of pharmaceutically acceptable carriers used in step (2) may be the same as those mentioned above in "1. Oral solid preparation according to the first embodiment of the present invention."

The mixing method in step (2) is not particularly limited as long as the milled aripiprazole hydrate crystal can be mixed with pharmaceutically acceptable carriers, such as excipients, binders, disintegrators, disintegration inhibitors, absorption enhancers, humectants, and adsorbents.

The powder of the aripiprazole hydrate crystal and the powder of a pharmaceutically acceptable carrier may be mixed by dry granulation, or may be mixed by wet granulation and formed into granules.

For wet granulation, various methods such as a fluidized bed granulation method, kneading granulation method, extrusion granulation method, and rotating granulation method can be used.

For example, when using a fluidized bed granulation method, granulating ingredients comprising various carriers are mixed by supplied air; while the granulating ingredients are continuously allowed to flow, a liquid is sprayed thereover to form granules. When using a kneading granulation method, granulating ingredients comprising various carriers are mixed by stirring; while the granulating ingredients are continuously stirred, a liquid is added thereto to form granules.

After the granulation, the obtained granules are, if necessary, passed through a screen to make the granules a desired size. The granules thus obtained are subjected to a drying process.

A wide variety of known methods can be used as the drying method. Examples of usable driers include fluidized bed dryers, fan dryers, vacuum dryers, and the like. For example, when using a fluidized bed dryer, the drying procedure is conducted with an air flow of 0.5 $m^3$/min to 50 $m^3$/min, and a supplied air temperature of 70 to 100°C for about 10 minutes to 1 hour.

The content of the aripiprazole hydrate crystal in the mixture obtained in step (2) is preferably set to the same range as mentioned above in "1. Oral solid preparation according to the first embodiment of the present invention."

When the oral solid preparation is formed into tablets by the method for producing the oral solid preparation of the present invention, the method further comprises a step of compressing the mixture obtained in step (2) into tablets.

Further adding a lubricant to the mixture obtained in step (2) is preferable in view of inhibiting the adhesion of the raw powder to a tableting machine during compression tableting in the subsequent step (3).

Various compression methods can be used in the compression tableting of step (3). Examples of usable methods include a wet granule compression method, direct powder compression method, dry granule compression method, and the like. Obtaining tablets by a direct powder compression method (direct compression method) is preferable in view of simple production of tablets that does not involve a complicated step of first preparing granules as in a wet granule compression method or dry granule compression method. Generally, when an oral solid preparation containing aripiprazole as an active ingredient is obtained by a direct compression method, low dissolubility tends to result. In contrast, in the present invention, because aripiprazole hydrate crystals as an active ingredient has a mean particle size within a specific range, a desired oral solid preparation comprising aripiprazole hydrate crystals, whose dissolubility is not reduced even when produced by a direct compression method, can be obtained.

When the oral solid preparation obtained by the production method of the present invention is formed into tablets, the tablets may be in the form of bilayer tablets, multilayer tablets, etc.

As long as high dissolubility, which is the effect of the present invention, is not impaired, a step of further coating may be performed, if necessary. For example, sugar-coated tablets, gelatin-coated tablets, enteric-coated tablets, and film-coated tablets can be produced.

The oral solid preparation obtained by the production method of the present invention exhibits the above-mentioned dissolution rate as measured by the dissolution test method described in "1. Oral solid preparation according to the first embodiment of the present invention."

3. Oral solid preparation according to the second embodiment of the present invention

The oral solid preparation according to the second embodiment of the present invention comprises, as an active ingredient, a highly hygroscopic aripiprazole anhydrous crystal having a mean particle size of 10 $\mu$m or less, and a pharmaceutically acceptable carrier.

The highly hygroscopic aripiprazole anhydrous crystals that can be used in the present invention are not particularly limited, and any crystals may be used. Examples of highly hygroscopic aripiprazole anhydrous crystals include the anhydrous crystals disclosed in Reference Examples 1 and 2 of Patent Document 2 (Japanese Patent No. 3760264), the aripiprazole anhydrous crystals disclosed in Patent Document 1 (JP1990-191256A), and aripiprazole Type I disclosed in Non-patent Document 1.

Further, the method for producing a highly hygroscopic aripiprazole anhydrous crystal is not particularly limited, and known production methods can be used. Examples of usable methods include a process disclosed in Example 1 of Patent Document 1 (JP1990-191256A), which comprises reacting 7-(4-bromobutoxy)-3,4-dihydrocarbostyril with 1-(2,3-dichlorophenyl)piperazine, and production processes disclosed in Patent Document 2 and Non-patent Document 1.

The highly hygroscopic aripiprazole anhydrous crystals are, for example, aripiprazole anhydrous crystals that have a hygroscopicity such that the water content after being allowed to stand in a desiccator at a temperature of 60°C and a humidity of 100% for 24 hours is more than 0.40%. The upper limit of the water content is preferably 4.5% or less, and more preferably 3.9% or less.

Upon exposure to moisture, highly hygroscopic conventional aripiprazole anhydrous crystals gain moisture, and convert into aripiprazole hydrates. Therefore, these crystals tended to be incapable of ensuring dissolubility with the lapse of time after production of the preparation. However, mean particle size reduction can provide a remarkable effect, i.e., ensuring a high dissolution rate of tablets even after being allowed to stand for a certain period of time in a dissolution test, in spite of the conversion of aripiprazole anhydrous into aripiprazole hydrate due to moisture.

The mean particle size of highly hygroscopic aripiprazole anhydrous crystals is about 10 $\mu$m or less, preferably 8 $\mu$m or less, and more preferably about 6 $\mu$m or less. Setting the mean particle size of highly hygroscopic aripiprazole anhydrous crystals to about 10 $\mu$m or less can provide an oral solid preparation that has high dissolubility immediately after production and also ensure dissolubility that is not impaired even after being allowed to stand for a certain period of time.

Setting the mean particle size to the above-mentioned specific range can achieve a dissolution rate of about 60% or more at pH 5 after 60 minutes (the dissolution rate that can maintain the maximum blood concentration ($C_{max}$)) in dissolution tests performed immediately after the production of the oral solid preparation, and after lapse of time.

The mean particle size of highly hygroscopic aripiprazole anhydrous crystals is not particularly limited. However, the mean particle size is preferably about 1 $\mu$m or more, more preferably about 2 $\mu$m or more, and most preferably about 2.5 $\mu$m or more, from the viewpoint of preventing an increase of the mean particle size due to reaggregation, reducing costs while not requiring excessive time to mill aripiprazole crystals, and facilitating handling. In particular, setting the mean particle size of aripiprazole anhydrous crystals to 2 $\mu$m or more, more preferably about 2.5 $\mu$m or more is preferable because it prevents dissolubility variation of aripiprazole anhydrous crystals as an active ingredient, and can stably provide an oral solid preparation with excellent dissolubility.

More specifically, the aripiprazole anhydrous crystals preferably have a mean particle size of about 1 to 10 $\mu$m (particularly about 2 to 10 $\mu$m, and more particularly about 2.5 to 10 $\mu$m), more preferably about 1 to 8 $\mu$m (particularly about 2 to 8 $\mu$m, and more particularly about 2.5 to 8 $\mu$m), and even more preferably about 1 to 6 $\mu$m (particularly about 2 to 6 $\mu$m, and more particularly about 2.5 to 6 $\mu$m).

The term "mean particle size" refers to volume mean diameter as calculated from the particle size distribution measured by a laser scattering particle size distribution analyzer.

The particle size ratio of the specific particle size of particles can be calculated from particle size distribution measured by a laser scattering particle size distribution analyzer.

The mean particle size measurement by the above method can also be performed using a light microscope. The measurement can be performed in accordance with the method described in the Japanese Pharmacopoeia (for example, B. General Tests, 3.04 Particle Size Determination, 1. First Optical Microscopy, described in the Handbook of the Japanese Pharmacopoeia, 16th Edition, Health, Labor and Welfare Ministry, Notification 65 (March 24, 2011)). The mean particle size measured according to the method described in the Japanese Pharmacopoeia may be, for example, in the range of 3 to 15 $\mu$m.

The oral solid preparation preferably comprises the aripiprazole crystal in an amount of about 0.5 to 80 wt.%, and more preferably about 1 to 70 wt.%.

Examples of pharmaceutically acceptable carriers in the oral solid preparation of the present invention include diluents and excipients generally used in pharmaceuticals, such as fillers, extenders, binders, humectants, disintegrators, surfactants, and lubricants.

The pharmaceutical composition of the present invention may be in the form of a general pharmaceutical preparation. Examples of such forms include tablets, flash-melt tablets, pills, powders, granules, capsules, and the like.

When the preparation is formed into tablets, a wide variety of carriers conventionally known in this field can be used. Examples of such carriers include excipients such as lactose, sucrose, sodium chloride, glucose, urea, starch, xylitol, mannitol, erythritol, sorbitol, calcium carbonate, kaolin, crystalline cellulose, and silicic acid; binders such as water, ethanol, propanol, simple syrup, glucose solution, starch solution, gelatin solution, carboxymethyl cellulose, shellac, methylcellulose, potassium phosphate, and polyvinylpyrrolidones; disintegrators such as dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, and lactose; disintegration inhibitors such as sucrose, stearin, cacao butter, and hydrogenated oils; absorption enhancers such as quaternary ammonium base and sodium lauryl sulfate; humectants such as glycerol and starch; adsorbents such as starch, lactose, kaolin, bentonite, and colloidal silica; and lubricants such as purified talc, stearate, boric acid powder, and polyethylene glycol. Tablets can also be formed as tablets with ordinary coatings, if necessary. Examples of such tables include sugar-coated tablets, gelatin-coated tablets, enteric-coated tablets, film-coated tablets, double-layer tablets, and multilayer tablets.

When the preparation is formed into pills, a wide variety of carriers conventionally known in this field can be used. Examples of usable carriers include excipients such as glucose, lactose, starch, cacao butter, hydrogenated vegetable oil, kaolin, and talc; binders such as powdered gum arabic, powdered tragacanth, gelatin, and ethanol; disintegrators such as laminaran and agar; and the like.

Capsules are prepared according to ordinary methods by mixing a highly hydroscopic aripiprazole anhydrous crystal with one or more of various carriers as exemplified above, and packing the mixture in hard gelatin capsules, soft capsules, hydroxypropylmethyl cellulose capsules (HPMC capsules), or the like.

If necessary, colorants, preservatives, perfumes, flavorings, sweeteners, and the like, as well as other drugs may be incorporated into the pharmaceutical composition.

When the solid oral preparation is formed into granules, a liquid (typically, water or an aqueous solution containing a binder) is added to a mixed powder of granulating ingredients comprising a highly hygroscopic aripiprazole anhydrous crystal and a carrier. As the carrier, various carriers known in this field can be used. Examples of such carriers include excipients, disintegrators, disintegration inhibitors, humectants, absorption enhancers, adsorbents, lubricants, colorants, and the like. Specific examples thereof include those mentioned above.

The oral solid preparation of the present invention may have any dissolution rate that is about 60% or more at pH 5 after 60 minutes as measured by a dissolution test method. When the dissolution rate at pH 5 after 60 minutes is about 60% or more, the active ingredient in the oral solid preparation administered can be sufficiently absorbed into the body, and the maximum blood concentration ($C_{max}$) can be maintained. Therefore, a dissolution rate of about 60% or more is preferable.

The dissolution test method is in accordance with the dissolution test method (paddle method) in the Japanese Pharmacopoeia, Sixteenth Edition, and is a method as described below.

One tablet of an oral solid preparation is added to 500 ml of acetate buffer (pH 5.0), which is used as a test liquid, thereby obtaining a test solution. Using the test solution, a dissolution test is performed at a paddle speed of 75 rpm in accordance with the method 2 (paddle method) for dissolution test. As the acetate buffer, a solution prepared by adding water to 1.97 g of acetic acid and 9.15 g of sodium acetate trihydrate to make 1000 ml of solution is used.

A standard solution is prepared in the following manner. 0.05 g of a standard sample of aripiprazole is weighed accurately, and ethanol (95%) is added to make exactly 50 ml of solution. Then, 6 ml of this solution is weighed accurately, and the test liquid is added to make exactly 1000 ml of solution.

The test solutions and the standard solution are subjected to filtration using a filter having a pore size of 10 or 20 $\mu$m. Each of the filtrates is introduced to a spectrophotometer equipped with a flow cell (cell length: 10 mm), and tested by UV visible absorbance spectroscopy. The differences between the absorbance of each solution at a wavelength of 249 nm and the absorbance thereof at a wavelength of 325 nm, which are At10, At20, At30, At45, At60, and As, are determined. At10, At20, At30, At45, and At60 indicate differences between the absorbances of each test solution at 10, 20, 30, 45, and 60 minutes after the beginning of the dissolution test, respectively. As indicates a difference between the absorbances of the standard solution.

The dissolution rate (%) relative to the indicated amount of aripiprazole is determined by the following formula:

```
Dissolution Rate (%) relative to the indicated amount of
aripiprazole = Amount of the aripiprazole standard product
(mg) x At x As x 2
```

In the Formula, As indicates a difference between the absorbances of the standard solution, and At indicates At10, At20, At30, At45, or At60.

More specifically, the measurement of the dissolution rate by the above dissolution test method can be performed by the method shown in Test Example 2 of the Examples below.

The method for administering the oral solid preparation of the present invention is not particularly limited. The preparation can be orally administered by a method suitable for the patient's age, sex, and other conditions (e.g., severity of disease).

The dosage of the oral solid preparation of the present invention can be suitably selected according to the dose regimen, the patient's age, sex, and other conditions (e.g., severity of disease), etc. Generally, the preparation is administered in an amount such that the amount of aripiprazole, which is an active ingredient, is about 0.1 to 10 mg per kg of body weight per day. The amount of aripiprazole per tablet of the oral solid preparation is in the range of about 1 to 100 mg, and preferably about 1 to 30 mg.

The oral solid preparation of the present invention is effective in the prevention and treatment of schizophrenia, including intractable (drug-resistant, chronic) schizophrenia with cognitive impairment and intractable (drug-resistant, chronic) schizophrenia without cognitive impairment; anxiety, including mild anxiety; mania, including acute mania such as bipolar disorder acute mania; bipolar disorder; depression, including bipolar disorder depression; autism, Down's syndrome, attention deficit hyperactivity disorder (ADHD), Alzheimer's disease, Parkinson's disease, and like neurodegenerative diseases; panic, obsessive compulsive disorder (OCD), sleep disorder, sexual dysfunction, alcohol and drug dependency, vomiting, motion sickness, obesity, migraine, cognitive impairment, and like central nervous system diseases; and major depression, behavioral and psychological symptoms of dementia (BPSD), Tourette's syndrome, bulimia nervosa, and chronic pain/fibromyalgia/chronic fatigue syndrome.

<u>4. Method for producing the oral solid preparation according to the second embodiment of the present invention</u>

The method for producing an oral solid preparation of the present invention comprises the steps of (1') milling a highly hygroscopic aripiprazole anhydrous crystal to obtain a finely milled crystal having a mean particle size of 10 $\mu$m or less, and (2') mixing the obtained finely milled crystal with a pharmaceutically acceptable carrier.

The finely milled crystal obtained by milling in step (1') can be directed subjected to the subsequent step (2'). For example, without drying after milling, the finely milled crystal can be used to produce the oral solid preparation.

Examples of the highly hygroscopic aripiprazole anhydrous crystal used in step (1') may be the same as the anhydrous crystals mentioned above in "3. Oral solid preparation according to the second embodiment of the present invention."

The method for milling the highly hygroscopic aripiprazole crystal in step (1') is not particularly limited, and may be wet milling or dry milling. Various milling methods and mills can be used. The milling step can be performed by suitably selecting or suitably combining the conditions, devices, and methods that can produce a highly hygroscopic aripiprazole anhydrous crystal having a mean particle size within the specific range of the present invention. From the viewpoint of ease of operation, dry milling is preferable.

When the milling in step (1') is performed by a dry milling method, examples of usable mills include jet mills, ball mills (e.g., Dyno mills), other low-energy mills (e.g., roller mills), and high-energy mills. Examples of high-energy mills include Netzsch mills, DC mills, planetary mills, and the like.

In particular, when a jet mill is used as a mill, the mean particle size of the milled highly hygroscopic aripiprazole anhydrous crystal can be set by two types of air pressure ("forcing pressure" for feeding the powder to the milling chamber, and "milling pressure" of air directly sent to the milling chamber) and the "feed rate of the powder." Generally, as the air pressure is set high and the feed rate of the powder is set low, milling strength increases and the particle size tends to be small.

When the milling in step (1') is performed by wet milling, usable mills include, for example, high-pressure homogenizers and bead mills.

Step (2') is a step of mixing the highly hygroscopic aripiprazole anhydrous crystal milled in step (1'), and a pharmaceutically acceptable carrier.

Examples of pharmaceutically acceptable carriers used in step (2') may be the same as those mentioned above in "3. Oral solid preparation according to the second embodiment of the present invention."

The mixing method in step (2') is not particularly limited as long as the milled highly hygroscopic aripiprazole anhydrous crystal can be mixed with pharmaceutically acceptable carriers, such as excipients, binders, disintegrators, disintegration inhibitors, absorption enhancers, humectants, and adsorbents.

The powder of the highly hygroscopic aripiprazole anhydrous crystal and the powder of a pharmaceutically acceptable carrier may be mixed by dry granulation, or may be mixed by wet granulation and formed into granules.

For wet granulation, various methods such as a fluidized bed granulation method, kneading granulation method, extrusion granulation method, and rotating granulation method can be used.

For example, when using a fluidized bed granulation method, granulating ingredients comprising various carriers are mixed by supplied air; while the granulating ingredients are continuously allowed to flow, a liquid is sprayed thereover to form granules. When using a kneading granulation method, granulating ingredients comprising various carriers are mixed by stirring; while the granulating ingredients are continuously stirred, a liquid is added thereto to form granules.

After the granulation, the obtained granules are, if necessary, passed through a screen to make the granules a desired size. The granules thus obtained are subjected to a drying process.

A wide variety of known methods can be used as the drying method. Examples of usable driers include fluidized bed dryers, fan dryers, vacuum dryers, and the like. For example, when using a fluidized bed dryer, the drying procedure is conducted with an air flow of 0.5 m$^3$/min to 50 m$^3$/min, and a supplied air temperature of 70 to 100°C for about 10 minutes to 1 hour.

The content of the highly hygroscopic aripiprazole anhydrous crystal in the mixture obtained in step (2') is preferably set to the same range as mentioned above in "3. Oral solid preparation according to the second embodiment of the present invention."

When the oral solid preparation is formed into tablets by the method for producing the oral solid preparation of the present invention, the method further comprises a step of compressing the mixture obtained in step (2') into tablets.

Further adding a lubricant to the mixture obtained in step (2') is preferable in view of inhibiting the adhesion of the raw powder to a tableting machine during compression tableting in the subsequent step (3').

Various compression methods can be used in the compression tableting of step (3'). Examples of usable methods include a wet granule compression method, direct powder compression method, dry granule compression method, and the like. Obtaining tablets by a direct powder compression method (direct compression method) is preferable in view of simple production of tablets that does not involve a complicated step of first preparing granules as in a wet granule compression method or dry granule compression method. Generally, when an oral solid preparation containing aripiprazole as an active ingredient is obtained by a direct compression method, low dissolubility tends to result. In contrast, in the present invention, because aripiprazole anhydrous crystal as an active ingredient has a mean particle size within a specific range, a desired oral solid preparation comprising aripiprazole anhydrous crystal, whose dissolubility is not reduced even when produced by a direct compression method, can be obtained.

When the oral solid preparation obtained by the production method of the present invention is formed into tablets, the tablets may be in the form of bilayer tablets, multilayer tablets, etc.

As long as high dissolubility, which is the effect of the present invention, is not impaired, a step of further coating may be performed, if necessary. For example, sugar-coated tablets, gelatin-coated tablets, enteric-coated tablets, and film-coated tablets can be produced.

The oral solid preparation obtained by the production method of the present invention exhibits the above-mentioned dissolution rate as measured by the dissolution test method described in "3. Oral solid preparation according to the second embodiment of the present invention."

Examples

The present invention is explained in detail below with reference to the Examples and Comparative Examples; however, the present invention is not limited thereto or thereby.

Examples and Comparative Examples of the first embodiment of the present invention shows as follows.

Aripiprazole hydrate crystals used in the Reference Examples and Examples shown below were prepared based on Japanese Patent Publication No. 3760264.

Reference Examples 1 to 3 and Reference Example 5 (Finely Milled Powder of Aripiprazole Hydrate Crystal)

A crystal of aripiprazole hydrate A prepared based on Example 1 of Japanese Patent Publication No. 3760264 was introduced into a jet mill (compact jet mill (JOM-min)), and subjected to dry mill by suitably adjusting air pressures (forcing pressure and milling pressure) and a feed rate so that the desired mean particle size was obtained. Milling was completed in 15 minutes, and a finely milled powder of aripiprazole hydrate crystal was obtained. The conditions of milling were suitably adjusted to obtain a desired mean particle size.

Reference Example 4 (Finely Milled Powder of Aripiprazole Hydrate Crystal)

Synthesis Example 1

Synthesis of Crude Aripiprazole

A 1.7-fold volume of purified water, 2.0-fold mol of potassium carbonate (anhydrous), and a 7.5-fold volume of ethanol were added to 7-hydroxy-3,4-dihydro-2(1H)-quinolinone (7-HDC) (standard amount), and the mixture was stirred at 40°C for about 0.5 hour. 1.1-fold mol of 4-chlorobutyl 2-nitrobenzene sulfonate (CBNBS) was added thereto and reacted at about 40°C for about 5 hours. After the solvent was distilled off, a 25-fold volume or more of purified water was added to the residue, followed by washing while stirring at about 50°C. The crystal was separated and washed with purified water.

A 14-fold volume of purified water, 1.07-fold mol of potassium carbonate (anhydrous), and 1.0 mol of 1-(2,3-dichlorophenyl)piperazine monohydrochloride(2,3-DCPP) were added to the obtained wet crystal, and the mixture was reacted at 80°C or more for about 4 hours. After cooling, the crystal was separated and washed with purified water. A 34-fold volume of ethyl acetate was added to the obtained wet crystal, and heated until solvent reflux to distill ethyl acetate and remove water. After cooling, ethyl acetate in an amount equivalent to the amount removed by distillation and 5 wt% of activated carbon were added thereto, and refluxed for 0.5 hour or more. The reaction mixture was hot-filtered to distill off a 20-fold volume of ethyl acetate, and then cooled. The crystal was separated, and dried at about 55°C to obtain crude aripiprazole.

The resulting crude aripiprazole (aripiprazole anhydrous crystal) was added to water-containing ethanol (ethanol: 80%, water: 20%), and heated to a solvent reflux temperature to completely dissolve the crude aripiprazole. The solution was cooled to precipitate an aripiprazole hydrate crystal, and the aripiprazole hydrate crystal was precipitated and milled by a wet pulverization device. After the solvent was removed by filtration, drying (airdrying overnight at room temperature) was performed to obtain an aripiprazole hydrate fine crystal (a crystal of aripiprazole hydrate A). The particle size was adjusted by wet pulverization operation conditions (suspension temperature, rotation speed, shape of wings for pulverization, etc.) to prepare a crystal of aripiprazole hydrate A having a desired particle size.

The particle size of the resulting finely milled powder of aripiprazole hydrate crystal was measured using Dry Unit 2000, which was a laser diffraction scattering particle size distribution analyzer (Laser Micron Sizer (LMS-2000e)), and the mean particle size was calculated from the obtained measurement results. Table 1 shows the measurement results.

The measurement conditions using a laser diffraction scattering particle size distribution analyzer were as follows: Particle refractive index: 1.750, imaginary part: 0.1, and dispersive pressure: 0.15 MPa.

Example 1 (Tablet Containing Finely Milled Powder of Aripiprazole Hydrate Crystal (3 mg per tablet))

The finely milled powder of aripiprazole hydrate crystal (9.3 g) (3 mg/tab calculated in terms of aripiprazole anhydride mol) obtained in Reference Example 1, directly compressible lactose (207.0 g), corn starch (30.0 g), crystalline cellulose (30.0 g), and hydroxypropyl cellulose (6.0 g) were introduced into a high speed granulator (kneader mini NSK-150, produced by Gokyo Seisakusyo), and mixed for about 10 minutes by rotating blades at about 500 rpm.

The mixture obtained above was introduced into a drum container rotating-type mixer, then magnesium stearate (3.0 g) was added thereto and mixed. After mixing, the mixture was supplied to a tableting machine (high-speed rotary tablet press, VIRGO, produced by Kikusui Seisakusho Ltd.), and formed into tablets each having a weight of 95.1 mg using a die punch.

Examples 2 to 5 (Tablet Containing Finely Milled Powder of Aripiprazole Hydrate Crystal (3 mg per tablet))

Tablets were obtained in the same manner as the method of Example 1, except that the finely milled powders obtained in Reference Examples 2 to 5 were used in place of the finely milled powder of aripiprazole hydrate crystal of Example 1.

Test Example 1 (Tablet Stability Test)

Each of the tablets of Examples 1 to 5 was allowed to stand under opening conditions: 40°C/75% RH for one week, two weeks, or one month. The dissolution rate was measured according to the following method.

Dissolution Test Method

An acetic acid buffer (500 ml) with a pH of 5.0 was used as a test liquid, and one tablet was introduced into the test liquid, thereby obtaining a test solution. Using the resulting test solution, the test was performed at 75 rpm according to the second paddle method of the dissolution test method. The acetic acid buffer mentioned above was prepared as 1000 ml of the acetic acid buffer by dissolving acetic acid (1.97 g) and sodium acetate trihydrate (9.15 g) in water.

The dissolution liquids obtained 10, 20, 30, 45, and 60 minutes after the beginning of the test were respectively referred to as Test Solution T10, T20, T30, T45, and T60.

As a standard solution, an aripiprazole standard product (0.05 g) was accurately weighed, and ethanol (95%) was added and dissolved therein to obtain exactly 50 ml of a solution. 6 ml of the solution was accurately weighed, and a test liquid was added thereto to obtain exactly 1000 ml of solution.

Each of the test solutions and standard solution was passed through a filter having a pore size of 10 or 20 $\mu$m. Each of the filtrates was introduced into a spectrophotometer equipped with a flow cell (cell length of 10 mm), and a test was performed according to an UV visible absorption spectrometry. As and At10, At20, At30, At45, and At60, which were differences between the absorbance at a wavelength of 249 nm and the absorbance at a wavelength of 325 nm, were individually measured.

Each of Test Solutions T10, T20, T30, and T45 after measurement was placed back into individual test containers. The same operation was performed for the other five samples.

As a dissolution device, any test device that applies to the paddle method harmonized trilaterally by the US, EU, and JP can be used; however, the device shown below was used. In Examples 1 to 3 and 5, Model: NTR-6100, produced by Toyama Sangyo Co., Ltd. was used. In Example 4, Model: NTR-6200A, produced by Toyama Sangyo Co., Ltd. was used.

The dissolution rate (%) relative to the indicated amount of aripiprazole was determined by the following formula.

```
Dissolution Rate (%) relative to the indicated amount of
aripiprazole = Amount of the aripiprazole standard product
(mg) x At x As x 2
```

Herein, As indicates a difference between the absorbances of the standard solution, and At indicates At10, At20, At30, At45, or At60.

Table 1 shows the dissolution rate of each sample obtained 60 minutes after the start of the dissolution test. One sample was not allowed to stand (initial value), and the other samples were allowed to stand under the opening conditions of 40°C/75% RH for one week, two weeks, or one month.

Table 1

| Example | | Ex. 1 | Ex. 2 | Ex. 3 | Ex. 4 | Ex. 5 |
|---|---|---|---|---|---|---|
| Aripiprazole crystal form | | Finely milled powder of hydrate | | | | |
| Mean particle size of aripiprazole ($\mu$m) | | Ref. Ex. 1 2.6 | Ref. Ex. 2 4.8 | Ref. Ex. 3 4.9 | Ref. Ex. 4 9.5 | Ref. Ex. 5 13.7 |
| Dissolution rate (%) Initial value | 60 min | 93.9 | 88.1 | 93.2 | 79.91 | 63.5 |
| Dissolution rate (%) 40°C/75% RH Opening conditions Stored for one week | 60 min | 92.0 | 86.4 | 88.9 | 76.17 | 61.8 |
| Dissolution rate (%) 40°C/75% RH Opening conditions Stored for two weeks | 60 min | 93.0 | 86.1 | 91.1 | 76.49 | 61.4 |
| Dissolution rate (%) 40°C/75% RH Opening conditions Stored for one month | 60 min | 91.9 | 87.9 | 90.3 | 77.12 | 64.7 |

Comparative Examples 1 to 3 (Tablet Containing Finely Milled Powder of Aripiprazole Hydrate Crystal (3 mg per tablet))

In the same manner as Reference Example 4, finely milled powders of aripiprazole hydrate crystals were prepared by milling so that the mean particle sizes shown in Table 2 were obtained (Reference Examples 6 to 8). Using the finely milled powders of aripiprazole hydrate crystals obtained in Reference Examples 6 to 8, tablets were prepared in the same manner as Example 1. The dissolution rate of each of the obtained tablets of Comparative Examples 1 to 3 was measured in the same manner as the stability test (dissolution test) of Example 4. Table 2 shows the dissolution rate.

Table 2

| Example | | Comp. Ex. 1 | Comp. Ex. 2 | Comp. Ex. 3 |
|---|---|---|---|---|
| Aripiprazole crystal form | | Finely milled powder of hydrate | | |
| Mean particle size of aripiprazole ($\mu$m) | | Ref. Ex. 6 15.1 | Ref. Ex. 7 20.0 | Ref. Ex. 8 29.6 |
| Dissolution rate (%) Initial value | 60 min | 55.79 | 49.61 | 35.97 |
| Dissolution rate (%) 40°C/75% RH Opening conditions Stored for one week | 60 min | 53.92 | 49.99 | 34.21 |
| Dissolution rate (%) 40°C/75% RH Opening conditions Stored for two weeks | 60 min | 52.93 | 47.85 | 34.30 |
| Dissolution rate (%) 40°C/75% RH Opening conditions Stored for one month | 60 min | 53.41 | 47.17 | 35.09 |

Example 6

Tablets were prepared in the same manner as Example 1, except that components of the tablets were changed to the following. A stability test (including a dissolution test) was performed in the same manner as Example 4. Table 3 shows the results of the stability test (dissolution test).

The jet-milled aripiprazole finely milled powder (hydrate crystal) (9.3 g) obtained in Reference Example 1, directly compressible lactose (114.0 g), crystalline cellulose (114.0 g), sodium carboxymethyl starch (21.0 g), carmellose calcium (21.0 g), and light anhydrous silicic acid (3.0 g) were introduced into a high speed granulator and mixed. Subsequently, magnesium stearate (3.0 g) was added thereto and mixed. The mixture was then supplied to a tableting machine, and formed into tablets using a die punch.

Table 3

| Example | | Example 6 |
|---|---|---|
| Finely milled aripiprazole crystal (hydrate) | | Reference Example 1 |
| Mean particle size of aripiprazole (μm) | | 2.6 |
| Dissolution rate (%) Initial value | 60 min | 90.60 |
| Dissolution rate (%) 40°C/75% RH Opening conditions Stored for one week | 60 min | 90.11 |
| Dissolution rate (%) 40°C/75% RH Opening conditions Stored for two weeks | 60 min | 90.69 |
| Dissolution rate (%) 40°C/75% RH Opening conditions Stored for one month | 60 min | 88.61 |

Examples and Comparative Examples of the second embodiment of the present invention shows as follows.

Synthesis Example 2

Synthesis of Crude Aripiprazole

A 1.7-fold volume of purified water, 2.0-fold mol of potassium carbonate (anhydrous), and a 7.5-fold volume of ethanol were added to 7-hydroxy-3,4-dihydro-2(1H)-quinolinone (7-HDC) (standard amount), and the mixture was stirred at 40°C for about 0.5 hour. 1.1-fold mol of 4-chlorobutyl 2-nitrobenzene sulfonate (CBNBS) was added thereto and reacted at about 40°C for about 5 hours. After the solvent was distilled off, a 25-fold volume or more of purified water was added to the residue, followed by washing while stirring at about 50°C. The crystal was separated and washed with purified water.

A 14-fold volume of purified water, 1.07-fold mol of potassium carbonate (anhydrous), and 1.0 mol of 1-(2,3-dichlorophenyl)piperazine monohydrochloride(2,3-DCPP) were added to the obtained wet crystal, and the mixture was reacted at 80°C or more for about 4 hours. After cooling, the crystal was separated and washed with purified water. A 34-fold volume of ethyl acetate was added to the obtained wet crystal, and heated until solvent reflux to distill ethyl acetate and remove water. After cooling, ethyl acetate in an amount equivalent to the amount removed by distillation and 5 wt% of activated carbon were added thereto, and refluxed for 0.5 hour or more. The reaction mixture was hot-filtered to distill off a 20-fold volume of ethyl acetate, and then cooled. The crystal was separated, and dried at about 55°C to obtain crude aripiprazole.

Recrystallization process

A 16-fold volume of ethanol and a 2-fold volume of purified water were added to the obtained crude aripiprazole, and heated until solvent reflux to completely dissolve the crude aripiprazole. A 2-fold volume of purified water was added thereto and heated again until reflux, and then stirred for 30 minutes or more under reflux. Subsequently, the resultant was cooled to 10°C or less to fully deposit a crystal, and then solid liquid separation was performed to obtain a crystal. The obtained crystal was dried at 80°C for 24 hours.

Milling Step

Aripiprazole obtained from recrystallization after drying was milled using a hammer mill for experiment (labo mill: Fuji Paudal Co., Ltd., model LM-05) under the following conditions.

Milling Conditions

Main axis rotation rate: 12000 rpm
Screen: 5 mmHB
Introduction speed: About 20 g/min

The properties of anhydrous crystal (Lot No. 1) synthesized in the Synthesis Example 2 were measured and evaluated according to the following method.

X-ray diffraction measurement

Using a X-ray diffractometer (D8 ADVANCE) produced by Bruker AXS, X-ray diffraction patterns in the range of a diffraction angle of 3 to 40° were measured according to a powder X-ray diffraction method. Copper was used as an anticathode.

Hygroscopic Test

About 1 g of aripiprazole obtained in the Synthesis Example 2 was weighed in each of the weighing bottles having a diameter of about 5 cm. Each of the bottles was sealed with Kimwipe, and allowed to stand in a desiccator including water. The desiccator was placed in an oven at 60°C, and allowed to stand for 24 hours.
The weighing bottles were removed from the desiccator, and allowed to stand for 16 hours or more in a desiccator (room temperature/about 30% RH) including an $MgCl_2/6H_2O$ saturated salt solution, thereby removing attached excess water. The water contents of the samples before and after the hygroscopic test were measured according to the following test procedure.

Water Content Measurement Method

0.1 g of the aripiprazole obtained in the Synthesis Example 2 and 0.1 g of the aripiprazole obtained after the hygroscopic test were accurately weighed. Using a moisture meter (CA-200) produced by Mitsubishi Chemical Analytech Co., Ltd., a test was performed according to the coulometric titration method, which is a water content measurement method (Karl Fischer method).

Melting Point Measurement

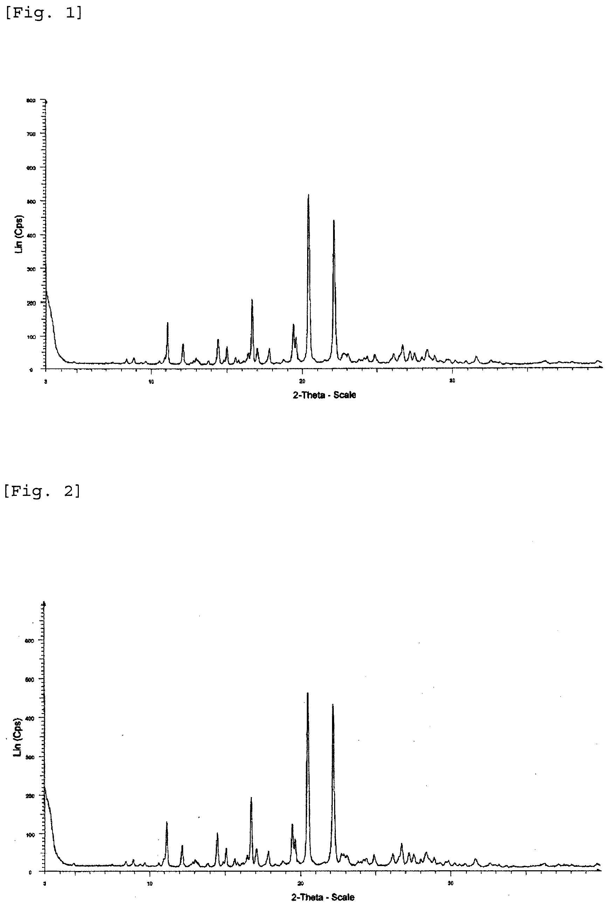

The melting point was measured by the following procedure using a melting point measurement apparatus (B-545) produced by BUCHI. A sample was introduced into a capillary tube, one end of which was closed, to a height of 2.5 to 3.5 mm from the bottom of the capillary tube. A block was heated to 130°C, and the capillary tube was introduced into the block. The block was heated at 3°C/min between 130 to 135°C and 1°C/min between 135°C to melting point, and the temperature at which the sample was completely dissolved was measured.
The properties of the anhydrous crystal (Lot No. 2) synthesized according to the same method as the method of the Synthesis Example 2 were measured in the same manner as above and evaluated.
Table 4 shows the measurement results. Figs. 1 (Lot No. 1) and 2 (Lot No. 2) show the X-ray diffraction pattern obtained by the X-ray diffraction measurement.

Table 4

| | Lot No. 1 | Lot No. 2 |
|---|---|---|
| Powder X-ray diffraction | Diffraction peak was recognized at $2\theta =$ 11.09°, 16.64°, 20.43°, 22.11° (Fig. 1) | Diffraction peak was recognized at $2\theta =$ 11.13°, 16.69°, 20.47°, 22.19° (Fig. 2) |
| Water content level (wt%) before humidification | 0.003 | 0.032 |
| Water content level (wt%) after humidification | 0.809 | 2.759 |
| Melting point (°C) | 139.7 | 139.8 |

By the measurements under the test conditions shown above, the formation of aripiprazole anhydrous Type I (highly Hygroscopic aripiprazole anhydrous crystal) was confirmed in the obtained crystals.

Reference Examples 9 to 12

Finely Milled Powder of Highly Hygroscopic Aripiprazole Anhydrous Crystal

Highly hygroscopic aripiprazole anhydrous crystals of Lot Nos. 1 and 2 obtained in the Synthesis Example 2 above were milled using a hummer mill (main axis rotation rate: 1200 rpm, screen: 5 mm herringbone, introduction speed: about 20 g/min). Then, dry milling was performed using a jet mill (spiral jet mill (spiral JM) or AO jet mill (AOJM)), air pressures (forcing pressure and milling pressure), and a feed rate shown in Table 5.

The particle size of the resulting finely milled powder of highly hygroscopic aripiprazole anhydrous crystal was measured using Dry Unit 2000, which was a laser diffraction scattering particle size distribution analyzer (Laser Micron Sizer (LMS-2000e)), and the mean particle size was calculated from the obtained measurement results. Table 5 shows the measurement results. The measurement conditions using a laser diffraction scattering particle size distribution analyzer were as follows: Particle refractive index: 1.750, imaginary part: 0.1, and dispersive pressure: 0.15 MPa.

The finely milled powder obtained above was evaluated by a hygroscopic test according to the following method.

About 1 g of milled powder was weighed in a weighing bottle with a diameter of 5 cm, allowed to stand in water/desiccator in which the temperature and humidity were respectively set to 60°C and 100%, and then allowed to stand in $MgCl_2 \cdot 6H_2O$ saturated salt solution/desiccator having a room temperature and humidity of about 30% for 24 hours. Thereafter, the water content was measured. The water content was measured according to the Karl Fischer method by accurately measuring 0.1 g of a sample.

Table 6 shows the measurement results. Each of the samples of finely milled powders measured showed 0.4% or more.

Table 5

| | Reference No. | Reference Example 9 | Reference Example 10 | Reference Example 11 | Reference Example 12 |
|---|---|---|---|---|---|
| | Milling material powder | Lot No. 1 | Lot No. 1 | Lot No. 2 | Lot No.2 |
| Milling conditions | Forcing pressure (MPa) | 0.6 | 0.6 | 0.4 | 0.4 |
| | Milling pressure (MPa) | 0.45 | 0.55 | 0.3 | 0.3 |
| | Feed rate (g/h) | 40 | 30 | 120 | 170 |
| | Jet mill used | Spiral JM | AO JM | AO JM | AO JM |
| | Mean particle size ($\mu$m) | 2.6 | 5.1 | 8.9 | 9.6 |

Examples 7 to 10

Method for Producing Tablets and Method for Stability Test (Containing Dissolution Test)

Tablets were formed using the finely milled powders prepared in Reference Examples 9 to 12 above.

The jet-milled aripiprazole finely milled powder (highly hygroscopic aripiprazole anhydrous crystal) (Type I) (9 g), directly compressible lactose (207 g), corn starch (30 g), crystalline cellulose (30 g), and hydroxypropyl cellulose (6 g) were introduced into a high speed granulator, and mixed for about 10 minutes by rotating blades at about 500 rpm. Thereafter, magnesium stearate was added in an amount of 1 wt% per tablet and mixed to prepare a powder for tableting. The powder for tableting was supplied to a tableting machine (Virgo 12HUK, produced by Kikusui Seisakusho Ltd.), and formed into tablets (tablets of Examples 7 to 10) each having a weight of 95 mg using a corner flat die punch having a diameter of 6.0 mm.

Test Example 2 (Tablet Stability Test)

Each of the tablets of Examples 7 to 10 was allowed to stand under opening conditions at 40°C/75% RH for one week, two weeks, or one month. The dissolution rate was measured according to the following method.

Dissolution Test Method

An acetic acid buffer (500 ml) with a pH of 5.0 was used as a test liquid, and one tablet was introduced into the test liquid, thereby obtaining a test solution. Using the resulting test solution, the test was performed at 75 rpm according to the second paddle method of the dissolution test method. The acetic acid buffer mentioned above was prepared as 1000 ml of the acetic acid buffer by dissolving acetic acid (1.97 g) and sodium acetate trihydrate (9.15 g) in water.

The dissolution liquids obtained 10, 20, 30, 45, and 60 minutes after the beginning of the test were respectively referred to as Test Solution T10, T20, T30, T45, and T60.

As a standard solution, an aripiprazole standard product (0.05 g) was accurately weighed, and ethanol (95%) was added and dissolved to obtain exactly 50 ml of a solution. 6 ml of the solution was accurately weighed, and a test liquid was added thereto to obtain exactly 1000 ml of solution.

Each of the test solutions and standard solution was passed through a filter having a pore size of 10 or 20 μm. Each of the filtrates was introduced into a spectrophotometer equipped with a flow cell (cell length of 10 mm), and a test was performed according to an UV visible absorption spectrometry. As and At10, At20, At30, At45, and At60, which were differences between the absorbance at a wavelength of 249 nm and the absorbance at a wavelength of 325 nm, were individually measured.

Each of the Test Solutions T10, T20, T30, and T45 after measurement was placed back into individual test containers. The same operation was performed for the other five samples.

As a dissolution device, any test device that applies to the paddle method harmonized trilaterally by the US, EU, and JP can be used; however, the device shown below was used. Model: NTR-6200A, produced by Toyama Sangyo Co., Ltd.

The dissolution rate (%) relative to the indicated amount of aripiprazole was determined by the following formula.

```
Dissolution Rate (%) relative to the indicated amount of
aripiprazole = Amount of the aripiprazole standard product
(mg) x At x As x 2
```

Herein, As indicates a difference between the absorbances of the standard solution, and At indicates At10, At20, At30, At45, or At60.

Table 6 shows the dissolution rate of each sample obtained 60 minutes after the start of the dissolution test. One sample was not allowed to stand (initial value), and the other samples were allowed to stand under the opening conditions of 40°C/75% RH for one week, two weeks, and one month.

Table 6

| Example | | Example 7 | Example 8 | Example 9 | Example 10 |
|---|---|---|---|---|---|
| Finely milled aripiprazole crystal | | Reference Example 9 | Reference Example 10 | Reference Example 11 | Reference Example 12 |
| Mean particle size of aripiprazole (μm) | | 2.6 | 5.1 | 8.9 | 9.6 |
| Hygroscopic test of finely milled aripiprazole crystal | | | | | |
| Water content level (wt%) | 0 hour | 0.10 | 0.21 | 0.24 | 0.29 |
| | 24 hours | 3.37 | 3.69 | 3.97 | 3.90 |
| Dissolution rate (%) Initial value | 60 min | 92.26 | 97.33 | 93.36 | 93.05 |
| Dissolution rate (%) 40°C/75% RH Opening conditions Stored for one week | 60 min | 88.09 | 88.99 | 77.78 | 76.09 |
| Dissolution rate (%) 40°C/75% RH Opening conditions Stored for two weeks | 60 min | 88.88 | 88.49 | 75.37 | 75.96 |

(continued)

| Example | | Example 7 | Example 8 | Example 9 | Example 10 |
|---|---|---|---|---|---|
| Finely milled aripiprazole crystal | | Reference Example 9 | Reference Example 10 | Reference Example 11 | Reference Example 12 |
| Dissolution rate (%) 40°C/75% RH Opening conditions Stored for one month | 60 min | 86.45 | 87.23 | 74.68 | 75.37 |

Comparative Example 4

Using the aripiprazole anhydrous crystal (Lot No. 1) milled with a hammer mill, the mean particle size was calculated in the same manner as Example 7. The mean particle size of the obtained finely milled powder was 27.2 $\mu$m. The hygroscopic test of the obtained finely milled powder was performed in the same manner as Example 7. As a result of the hygroscopic test, the water content at 0 hour was 0.00 wt%; however, the water content after 24 hours was 0.49 wt%.
Using the crystal of the obtained finely milled powder, tablets were produced in the same manner as Example 7. The stability test of the obtained tablets was performed in the same manner as in the above Examples. The results indicated that the dissolution rate (%) was 78.67% as the initial value, 42.66% after one week of storage, 41.04% after two weeks of storage, and 41.75% after one month of storage.

Example 11

Tablets were prepared in the same manner as Example 7, except that the components of the tablets were changed to the following. A stability test (including a dissolution test) was performed. Table 7 shows the results of the stability test (dissolution test).
The jet-milled aripiprazole finely milled powder (highly hygroscopic aripiprazole anhydrous crystal) (Type I) (9 g) obtained in Reference Example 9, directly compressible lactose (114.0 g), crystalline cellulose (114.0 g), sodium carboxymethyl starch (21.0 g), carmellose calcium (21.0 g), and light anhydrous silicic acid (3.0 g) were mixed, and then magnesium stearate (3.0 g) was added thereto and mixed. The mixture was then supplied to a tableting machine, and formed into tablets using a die punch.

Table 7

| Example | | Example 11 |
|---|---|---|
| Finely milled aripiprazole crystal | | Reference Example 9 |
| Mean particle size of aripiprazole ($\mu$m) | | 2.6 |
| Hygroscopic test of finely milled aripiprazole crystal | | |
| Water content level (wt%) | 0 hour<br>24 hours | 0.10<br>3.37 |
| Dissolution rate (%) Initial value | 60 min | 77.46 |
| Dissolution rate (%) 40°C/75% RH Opening conditions Stored for one week | 60 min | 75.40 |
| Dissolution rate (%) 40°C/75% RH Opening conditions Stored for two weeks | 60 min | 76.09 |
| Dissolution rate (%) 40°C/75% RH Opening conditions Stored for one month | 60 min | 75.74 |

**Claims**

1. An oral solid preparation comprising, as an active ingredient, a finely milled powder obtained by milling a highly hygroscopic aripiprazole anhydrous crystal, which has a hygroscopicity such that the water content after being allowed to stand in a desiccator at a temperature of 60°C and a humidity of 100% for 24 hours is more than 0.40%, and a pharmaceutically acceptable carrier, the finely milled crystal having a mean particle size of 1 to 10 $\mu$m, wherein the mean particle size is the volume mean diameter as calculated from the particle size distribution measured by a laser scattering particle size distribution analyzer.

2. The oral solid preparation according to claim 1, wherein the finely milled powder has a mean particle size of 1 to 8 $\mu$m.

3. The oral solid preparation according to claim 1, wherein the finely milled powder has a mean particle size of 1 to 6 $\mu$m.

4. The oral solid preparation according to any one of claims 1 to 3, wherein the finely milled powder has a mean particle size of 2.5 $\mu$m or more.

5. The oral solid preparation according to any one of claims 1 to 4, in the form of a tablet, pill, granule or capsule.

6. A method for producing an oral solid preparation comprising the steps of:

(1') milling a highly hygroscopic aripiprazole anhydrous crystal, which has a hygroscopicity such that the water content after being allowed to stand in a desiccator at a temperature of 60°C and a humidity of 100% for 24 hours is more than 0.40%, into a finely milled powder having a mean particle size of 1 to 10 $\mu$m, wherein the mean particle size is the volume mean diameter as calculated from the particle size distribution measured by a laser scattering particle size distribution analyzer; and
(2') mixing the obtained finely milled powder with a pharmaceutically acceptable carrier.

7. The method for producing an oral solid preparation according to claim 6, wherein the oral solid preparation is a tablet, and the method further comprises the step of (3') compressing the mixture obtained in step (2') into a tablet.

[Fig. 1]

[Fig. 2]

Europäisches
Patentamt

European
Patent Office

Office européen
des brevets

## EUROPEAN SEARCH REPORT

Application Number

EP 17 15 6237

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/000392 A1 (SHANGHAI ZHONGXI PHARMACEUTICAL CORP [CN]; SHANGHAI ZHONGXI SUNVE PHAR) 3 January 2013 (2013-01-03) * effect examples 2-5; claims 25-30 * | 1-7 | INV. A61K9/14 A61K9/20 |
| X,P | -& GB 2 505 859 A (SHANGHAI ZHONGXI PHARMACEUTICAL CORP [CN]) 12 March 2014 (2014-03-12) * effect examples 2-5 on page 27-29; claims 25-30 * | 1-7 | |
| X | US 2007/272777 A1 (SAMBURSKI GUY [IL] ET AL) 29 November 2007 (2007-11-29) * claim 18; examples 1-4 * | 1-7 | |
| X | WO 2005/041937 A2 (OTSUKA PHARMA CO LTD [JP]; KOSTANSKI JANUSZ W [US]; MATSUDA TAKAKUNI []) 12 May 2005 (2005-05-12) * page 13, lines 21-32; claims 17,18; examples 1-3 * | 1-7 | TECHNICAL FIELDS SEARCHED (IPC) |
| A,D | WO 03/026659 A1 (OTSUKA PHARMA CO LTD [JP]; BANDO TAKUJI [JP]; AOKI SATOSHI [JP]; KAWAS) 3 April 2003 (2003-04-03) * pages 25,67; example 1 * * page 12, lines 4-26 * | 1-7 | A61K |

The present search report has been drawn up for all claims

| Place of search | Date of completion of the search | Examiner |
|---|---|---|
| The Hague | 2 May 2017 | Konter, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 15 6237

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2017

| Patent document cited in search report | | Publication date | Patent family member(s) | | Publication date |
|---|---|---|---|---|---|
| WO 2013000392 | A1 | 03-01-2013 | CN | 102850268 A | 02-01-2013 |
| | | | GB | 2505859 A | 12-03-2014 |
| | | | IL | 230190 A | 31-08-2016 |
| | | | JP | 6014130 B2 | 25-10-2016 |
| | | | JP | 2014518224 A | 28-07-2014 |
| | | | US | 2014135344 A1 | 15-05-2014 |
| | | | WO | 2013000392 A1 | 03-01-2013 |
| GB 2505859 | A | 12-03-2014 | CN | 102850268 A | 02-01-2013 |
| | | | GB | 2505859 A | 12-03-2014 |
| | | | IL | 230190 A | 31-08-2016 |
| | | | JP | 6014130 B2 | 25-10-2016 |
| | | | JP | 2014518224 A | 28-07-2014 |
| | | | US | 2014135344 A1 | 15-05-2014 |
| | | | WO | 2013000392 A1 | 03-01-2013 |
| US 2007272777 | A1 | 29-11-2007 | BR | PI0608185 A2 | 17-11-2009 |
| | | | EP | 1919453 A2 | 14-05-2008 |
| | | | JP | 2008537540 A | 18-09-2008 |
| | | | TW | 200800202 A | 01-01-2008 |
| | | | US | 2007272777 A1 | 29-11-2007 |
| | | | WO | 2007075871 A2 | 05-07-2007 |
| WO 2005041937 | A2 | 12-05-2005 | AR | 046141 A1 | 23-11-2005 |
| | | | AT | 411797 T | 15-11-2008 |
| | | | AU | 2004285448 A1 | 12-05-2005 |
| | | | BR | PI0415531 A | 26-12-2006 |
| | | | CA | 2543242 A1 | 12-05-2005 |
| | | | CN | 1870980 A | 29-11-2006 |
| | | | CO | 5690539 A2 | 31-10-2006 |
| | | | CY | 1109040 T1 | 02-07-2014 |
| | | | DK | 1675573 T3 | 05-01-2009 |
| | | | EC | SP066512 A | 10-10-2006 |
| | | | EC | SP12006512 A | 29-02-2012 |
| | | | EP | 1675573 A2 | 05-07-2006 |
| | | | ES | 2315721 T3 | 01-04-2009 |
| | | | GE | P20115205 B | 26-04-2011 |
| | | | HK | 1095266 A1 | 19-11-2010 |
| | | | IL | 175072 A | 30-06-2015 |
| | | | JP | 4836797 B2 | 14-12-2011 |
| | | | JP | 5372032 B2 | 18-12-2013 |
| | | | JP | 2007509148 A | 12-04-2007 |
| | | | JP | 2011102316 A | 26-05-2011 |
| | | | KR | 20060118450 A | 23-11-2006 |
| | | | LU | 92427 I2 | 11-06-2014 |
| | | | MX | PA06004489 A | 20-06-2006 |

EPO FORM P0459

For more details about this annex : see Official Journal of the European Patent Office, No. 12/82

page 1 of 4

## ANNEX TO THE EUROPEAN SEARCH REPORT
## ON EUROPEAN PATENT APPLICATION NO.

EP 17 15 6237

This annex lists the patent family members relating to the patent documents cited in  the above-mentioned European search report.
The members are as contained in the European Patent Office EDP file on
The European Patent Office is in no way liable for these particulars which are merely given for the purpose of information.

02-05-2017

| Patent document cited in search report | Publication date | Patent family member(s) | | Publication date |
|---|---|---|---|---|
| | | MY | 146093 A | 29-06-2012 |
| | | NO | 334919 B1 | 14-07-2014 |
| | | NO | 2014027 I1 | 10-11-2014 |
| | | NZ | 546063 A | 31-05-2009 |
| | | PE | 08112005 A1 | 25-09-2005 |
| | | PT | 1675573 E | 30-12-2008 |
| | | RU | 2342927 C2 | 10-01-2009 |
| | | SI | 1675573 T1 | 28-02-2009 |
| | | TW | I383810 B | 01-02-2013 |
| | | TW | 201204413 A | 01-02-2012 |
| | | UA | 82561 C2 | 25-04-2008 |
| | | US | 2005148597 A1 | 07-07-2005 |
| | | US | 2008107745 A1 | 08-05-2008 |
| | | US | 2008112985 A1 | 15-05-2008 |
| | | US | 2008112986 A1 | 15-05-2008 |
| | | US | 2008221121 A1 | 11-09-2008 |
| | | US | 2012100190 A1 | 26-04-2012 |
| | | US | 2014018369 A1 | 16-01-2014 |
| | | US | 2015024056 A1 | 22-01-2015 |
| | | WO | 2005041937 A2 | 12-05-2005 |
| | | ZA | 200602347 B | 26-09-2007 |
| WO 03026659 A1 | 03-04-2003 | AR | 033485 A1 | 26-12-2003 |
| | | AR | 056503 A2 | 10-10-2007 |
| | | AT | 322269 T | 15-04-2006 |
| | | AT | 464050 T | 15-04-2010 |
| | | AT | 465736 T | 15-05-2010 |
| | | AT | 465737 T | 15-05-2010 |
| | | AT | 467416 T | 15-05-2010 |
| | | AU | 2002334413 B2 | 04-11-2004 |
| | | BR | 0205391 A | 29-07-2003 |
| | | CA | 2426921 A1 | 03-04-2003 |
| | | CA | 2688860 A1 | 03-04-2003 |
| | | CA | 2688915 A1 | 03-04-2003 |
| | | CA | 2688934 A1 | 03-04-2003 |
| | | CA | 2689051 A1 | 03-04-2003 |
| | | CA | 2689052 A1 | 03-04-2003 |
| | | CN | 1463191 A | 24-12-2003 |
| | | CN | 1699346 A | 23-11-2005 |
| | | CN | 1817882 A | 16-08-2006 |
| | | CN | 101423492 A | 06-05-2009 |
| | | CN | 101423493 A | 06-05-2009 |
| | | CN | 101434573 A | 20-05-2009 |
| | | CN | 101434574 A | 20-05-2009 |
| | | CN | 101434575 A | 20-05-2009 |
| | | CN | 101574347 A | 11-11-2009 |

EPO FORM P0459

For more details about this annex : see Official Journal of the European Patent Office, No. 12/82

# EP 3 187 173 A1

## ANNEX TO THE EUROPEAN SEARCH REPORT
## ON EUROPEAN PATENT APPLICATION NO.

EP 17 15 6237

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2017

| Patent document cited in search report | Publication date | Patent family member(s) | | Publication date |
|---|---|---|---|---|
| | | CN | 101574348 A | 11-11-2009 |
| | | CN | 101579343 A | 18-11-2009 |
| | | CN | 101579344 A | 18-11-2009 |
| | | CN | 101792415 A | 04-08-2010 |
| | | CN | 106420627 A | 22-02-2017 |
| | | CN | 106420640 A | 22-02-2017 |
| | | DE | 60210409 T2 | 16-11-2006 |
| | | DK | 1330249 T3 | 07-08-2006 |
| | | DK | 1419776 T3 | 05-07-2010 |
| | | DK | 1927355 T3 | 19-07-2010 |
| | | DK | 1927356 T3 | 26-07-2010 |
| | | DK | 1927357 T3 | 16-08-2010 |
| | | EP | 1330249 A1 | 30-07-2003 |
| | | EP | 1419776 A2 | 19-05-2004 |
| | | EP | 1925308 A1 | 28-05-2008 |
| | | EP | 1927355 A1 | 04-06-2008 |
| | | EP | 1927356 A1 | 04-06-2008 |
| | | EP | 1927357 A2 | 04-06-2008 |
| | | ES | 2261750 T3 | 16-11-2006 |
| | | ES | 2343179 T3 | 26-07-2010 |
| | | ES | 2343219 T3 | 26-07-2010 |
| | | ES | 2343220 T3 | 26-07-2010 |
| | | ES | 2343602 T3 | 04-08-2010 |
| | | IL | 153838 A | 13-04-2008 |
| | | JP | 3750023 B2 | 01-03-2006 |
| | | JP | 3760264 B2 | 29-03-2006 |
| | | JP | 4614870 B2 | 19-01-2011 |
| | | JP | 2003212852 A | 30-07-2003 |
| | | JP | 2004256555 A | 16-09-2004 |
| | | JP | 2006070045 A | 16-03-2006 |
| | | MX | PA03000440 A | 06-10-2003 |
| | | NO | 328134 B1 | 14-12-2009 |
| | | NO | 336262 B1 | 06-07-2015 |
| | | NO | 336263 B1 | 06-07-2015 |
| | | NO | 336264 B1 | 06-07-2015 |
| | | NO | 336265 B1 | 06-07-2015 |
| | | NO | 336679 B1 | 19-10-2015 |
| | | PE | 01242009 A1 | 07-03-2009 |
| | | PL | 360900 A1 | 20-09-2004 |
| | | PT | 1330249 E | 30-06-2006 |
| | | PT | 1419776 E | 28-04-2010 |
| | | PT | 1927355 E | 11-06-2010 |
| | | PT | 1927356 E | 24-05-2010 |
| | | PT | 1927357 E | 08-06-2010 |
| | | SI | 1330249 T1 | 31-10-2006 |
| | | SI | 1419776 T1 | 30-07-2010 |

EPO FORM P0459

For more details about this annex : see Official Journal of the European Patent Office, No. 12/82

page 3 of 4

**ANNEX TO THE EUROPEAN SEARCH REPORT
ON EUROPEAN PATENT APPLICATION NO.**

EP 17 15 6237

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2017

| Patent document cited in search report | Publication date | Patent family member(s) | Publication date |
|---|---|---|---|
| | | SI 1927355 T1 | 30-07-2010 |
| | | SI 1927356 T1 | 30-07-2010 |
| | | UA 84764 C2 | 25-11-2008 |
| | | US 2004058935 A1 | 25-03-2004 |
| | | US 2007202181 A1 | 30-08-2007 |
| | | US 2007203150 A1 | 30-08-2007 |
| | | US 2007203151 A1 | 30-08-2007 |
| | | US 2007203152 A1 | 30-08-2007 |
| | | US 2007212421 A1 | 13-09-2007 |
| | | US 2007213343 A1 | 13-09-2007 |
| | | US 2007213344 A1 | 13-09-2007 |
| | | US 2012000998 A1 | 05-01-2012 |
| | | US 2012016123 A1 | 19-01-2012 |
| | | US 2012315302 A1 | 13-12-2012 |
| | | US 2012316179 A1 | 13-12-2012 |
| | | US 2012316180 A1 | 13-12-2012 |
| | | US 2014030523 A1 | 30-01-2014 |
| | | US 2014309236 A1 | 16-10-2014 |
| | | US 2015225347 A1 | 13-08-2015 |
| | | US 2016251315 A1 | 01-09-2016 |
| | | WO 03026659 A1 | 03-04-2003 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2191256 A
- JP 2608788 B
- JP 3760264 B

### Non-patent literature cited in the description

- **SATOSHI AOKI et al.** Study on Crystal Transformation of Aripiprazole. *Proceedings of the Fourth Japan-Korea Joint Symposium on Separation Technique,* 06 October 1996, 937-940
- Handbook of the Japanese Pharmacopoeia. 24 March 2011